(19) Europäisches Patentamt
European Patent Office
Office européen des brevets

(11) Veröffentlichungsnummer : **0 011 598**
**B1**

(12) **EUROPÄISCHE PATENTSCHRIFT**

(45) Veröffentlichungstag der Patentschrift : **26.01.83**

(51) Int. Cl.³ : **C 07 D307/81**, C 07 D307/80, C 07 C 93/14, A 61 K 31/135, A 61 K 31/34

(21) Anmeldenummer : **79810118.4**

(22) Anmeldetag : **08.10.79**

(54) **Verfahren zur Herstellung von Derivaten von Dimeren des Isoeugenols.**

(30) Priorität : **15.11.78 AT 8151/78**

(43) Veröffentlichungstag der Anmeldung : **28.05.80 Patentblatt 80/11**

(45) Bekanntmachung des Hinweises auf die Patenterteilung : **26.01.83 Patentblatt 83/04**

(84) Benannte Vertragsstaaten : **BE CH DE FR GB IT LU NL SE**

(56) Entgegenhaltungen :
**DE A 2 543 099**
**DE A 2 812 664**
**DE B 1 138 042**

**Ehrhart Ruschig, Arzneimittel Entwicklung Wirkung Darstellung, Band 3, Verlag Chemie Weinheim/Bergstr. 1972.**

(73) Patentinhaber : **PLC PHARMACEUTICAL LICENCES COMPANY LTD.**
**7, Chemin de Trembley**
**CH-1197 Prangins VD (CH)**

(72) Erfinder : **Griengl, Herfried**
**Argenot-Strasse 23**
**A-8047 Graz (AT)**
Erfinder : **Foidl, Gabriele**
**Dr. Robert-Graf-Strasse 10**
**A-8010 Graz (AT)**

(74) Vertreter : **Meyer, Hans**
**15, rue Viollier**
**CH-1207 Genève (CH)**

**EP 0 011 598 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von Derivaten von Dimeren des Isoeugenols

Die Erfindung betrifft ein Verfahren zur Herstellung zum Teil neuer wasserlöslicher Derivate von Dimeren des Isoeugenols. (E)-Isoeugenol geht bei milder Oxidation, beispielsweise mit Eisen (III) chlorid, in ein Dimeres, trans-2,3-Dihydro-2-(4-hydroxy-3-methoxyphenyl)-7-methoxy-3-methyl-5-(E)-propenylbenzofuran (« Dehydrodiisoeugenol ») über, wie es erstmals von H. Erdtman in der Biochemischen Zeitschrift im Band 258 aus dem Jahre 1933 auf den Seiten 172 bis 180 beschrieben wurde.

In saurem Medium wird hingegen ein Dimeres anderer Struktur, nämlich (1α, 2β, 3α)-1-Aethyl-3-(4-hydroxy-3-methoxyphenyl)-6-methoxy-2-methyl-5-indanol (« Diisoeugenol ») gebildet. Dies wurde erstmals von E. Puxeddu beobachtet und in der Gazzetta Chimica Italiana im Jahr 1909 auf den Seiten 131 bis 137 veröffentlicht.

Beide Dimeren besitzen wertvolle Eigenschaften als Wirkstoffe von Therapeutika für Lebererkrankungen, wie nachstehend beispielsweise für « Dehydrodiisoeugenol » ausgeführt ist :

Ein Standard-Prüfverfahren auf pharmakologische Wirkung bei experimenteller Leberschädigung ist dabei der Hexobarbital-Schlaftest, dessen Durchführung u.a. in einer Arbeit von G. Vogel et al., erschiener in Arzneimittelforschung, Band 25 (1975), auf den Seiten 82 bis 89 und 179 bis 188 beschrieben ist. Dazu erhielten Ratten in Gruppen zu je 15 Tieren 100 mg/kg der zu testenden Verbindung in Carboxymethylcellulose intraperitoneal und 90 min später 0,3 ml/kg Tetrachlorkohlenstoff in Olivenöl mittels Schlundsonde. 48 h später wurden die Ratten durch intraperitoneale Verabreichung von 70 mg/kg Hexobarbital narkotisiert und es wurde die Schlafdauer bestimmt :

| | min |
|---|---|
| Tetrachlorkohlenstoff ohne Testverbindung | 128 |
| Kontrolle (nur Hexobarbital) | 83 |
| trans-2,3-Dihydro-2-(4-hydroxy-3-methoxyphenyl-7-methoxy-3-methyl-5-(E)-propenylbenzofuran (« Dehydrodiisoeugenol ») | 97 |
| Silymarin | 115 |

Die Wirkung von trans-2,3-Dihydro-2-(4-hydroxy-3-methoxyphenyl)-7-methoxy-3-methyl-5-(E)-propenylbenzofuran (« Dehydrodiisoeugenol ») ist hochsignifikant ($p < 0{,}01$), jene der Vergleichsverbindung Silymarin, ein bekanntes Lebertherapeutikum, statistisch nicht signifikant.

Ein grosser Nachteil in Bezug auf die Applikationsbreite der angeführten Dimeren des Isoeugenols ist jedoch ihre geringe Wasserlöslichkeit. Es gelingt zwar, diese Dimeren mit Formaldehyd und einem sekundären Amin auf übliche Art in die entsprechenden Mannich-Basen überzuführen, jedoch sind die Ausbeuten schlecht und es ist schwierig, unumgesetzten Formaldehyd aus dem Reaktionsansatz abzutrennen.

Ziel der Erfindung ist es, gut wasserlösliche Derivate von Dimeren des Isoeugenols zu schaffen, die in guter Ausbeute hergestellt werden können und in ihrer pharmakologischen Wirkung den Ausgangsstoffen entsprechen. Dieses Ziel wird durch die Erfindung erreicht.

Gegenstand der Erfindung ist ein neues Verfahren, das es gestattet, N-Alkyl-N-(2-hydroxyalkyl)-aminomethylderivate von Dimeren des Isoeugenols in ausgezeichneter Ausbeute bei einfacher Reaktionsführung darzustellen. Das Prinzip des neuen Verfahrens besteht darin, dass man ein Dimeres von Isoeugenol der Formel

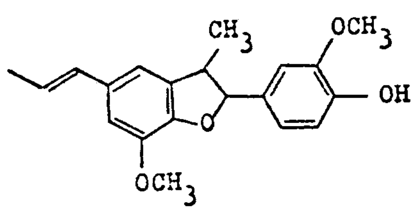

(I, Dehydrodiisoeugenol)

oder der Formel

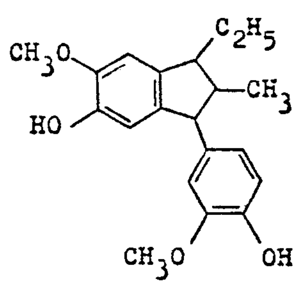

(II, Diisoeugenol)

mit einem Oxazolidin der allgemeinen Formel (III) umsetzt

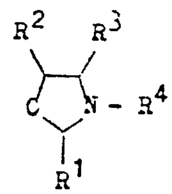

(III)

worin

$R^1$ vorzugsweise für Wasserstoff, jedoch auch für einen verzweigten oder unverzweigten Alkylrest mit ein bis vier Kohlenstoffatomen, insbesondere eine Isopropylgruppe, jedoch nicht eine tert. Butylgruppe, oder einen gegebenenfalls durch Halogen, insbesondere Chlor substituierten, vorzugsweise jedoch unsubstituierten Phenylrest,

$R^2$ für Wasserstoff oder einen Phenylrest,

$R^3$ für Wasserstoff oder eine Methylgruppe,

$R^4$ für eine verzweigte oder unverzweigte Alkylgruppe mit ein bis vier Kohlenstoffatomen, die als Substituenten eine Hydroxygruppe tragen kann, vorzugsweise eine Methyl-, Aethyl-, 2-Hydroxyäthyl-, Propyl-, Isopropyl-, Butyl- oder tert. Butylgruppe, eine Allylgruppe oder eine Benzylgruppe steht, wobei Verbindungen der allgemeinen Formel (IV)

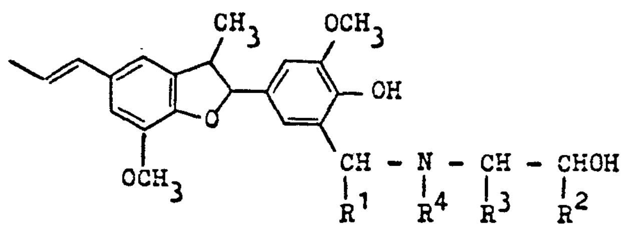

(IV)

bzw. Verbindungen der allgemeinen Formel V

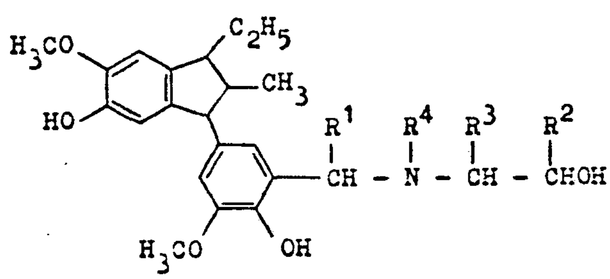

(V)

entstehen, worin $R^1$, $R^2$, $R^3$, $R^4$ die gleiche Bedeutung wie in Verbindungen der allgemeinen Formel (III) haben.

Durch Ueberführung der nach dem erfindungsgemässen Verfahren hergestellten Verbindungen der allgemeinen Formel (IV) und (V) mit Säuren in die entsprechenden Ammoniumsalze, wie weiter unten noch ausgeführt wird, gelingt es, ausgezeichnete Wasserlöslichkeit zu erzielen, wobei die Anwesenheit der neueingeführten hydrophilen alkoholischen Hydroxylgruppe ebenfalls einen günstigen Effekt zeigt.

Die Herstellung der für die Umsetzung benötigten 1,3-Oxazolidine der allgemeinen Formel (III) erfolgt auf an und für sich übliche Art durch Kondensation von Aldehyden der allgemeinen Formel $R^1$—CHO mit N-substituierten Aethanolaminderivaten der allgemeinen Formel $\underset{R^4}{HN}-\underset{R^3}{CH}-\underset{R^2}{CHOH}$, worin $R^1$, $R^2$, $R^3$, $R^4$ die gleiche Bedeutung wie in den Verbindungen der allgemeinen Formel (III) haben und wie dies beispielsweise von E.D. Bergmann, E. Zimkin und S. Pinchas in « Recueil des Travaux Chimiques des Pays-Bas » im Band 71 (1952) auf der Seite 171 beschrieben ist.

Zur experimentellen Durchführung des erfindungsgemässen Verfahrens wählt man vorzugsweise äquimolare Mengen « Dehydrodiisoeugenol » oder « Diisoeugenol » und 1,3-Oxazolidin oder einen Ueberschuss des letzteren und lässt in einem geeigneten Lösungsmittel bei Temperaturen zwischen Raumtemperatur und der Siedetemperatur des eingesetzten Lösungsmittels, z.B. bei 40 bis 80 °C, einige Stunden bis einige Tage, vorzugsweise 10 bis 60 Stunden reagieren.

Als Lösungsmittel kommen in Frage Alkohole wie Methanol, Aethanol oder 2-Propanol, Aether wie Diäthyläther, Diisopropyläther, Dioxan oder Tetrahydrofuran oder Nitrile wie Acetonitril oder Propionitril.

Die Aufarbeitung erfolgt in der Regel durch Verdampfen des Lösungsmittels im Vakuum und Kristallisation oder Umfällung des Rückstands, gegebenenfalls unter Vorschaltung einer chromatographischen Reinigungsoperation.

Die erhaltenen freien Basen der Formeln (IV) und (V) können in ihre Ammoniumsalze, insbesondere ihre Salze mit physiologisch annehmbaren Säuren übergeführt werden. Als solche Säuren eignen sich vorzugsweise anorganische Säuren, wie Chlorwasserstoff-, Bromwasserstoff-, Schwefel- oder Phosphorsäure, oder organische Säuren wie Ameisen-, Essig-, Propion-, Bernstein-, Glykol-, Milch-, Apfel-, Wein-, Zitronen-, Aethansulfon-, Hydroxyäthansulfon-, Ascorbin-, Lipon-, Asparagin-, α-Ketoglutar-, Glutamin-, Zucker-, Glucon-, Schleim- oder Thiazolidincarbonsäure. Die Bildung der Salze erfolgt z.B. durch Umsetzung der Säure mit der freien Base der Formel (IV) bzw. (V), zweckmässig in einem geeigneten Lösungsmittel, wie einem der oben angegebenen.

Infolge des engen Zusammenhangs zwischen den freien Verbindungen und den Salzen sind im folgenden bei Nennung der einen jeweils sinngemäss auch die anderen zu verstehen.

Die nach dem erfindungsgemässen Verfahren hergestellten und zum Teil neuen Verbindungen besitzen wertvolle pharmakologische Eigenschaften, insbesondere eine Schutzwirkung gegen Leberschäden. So zeigen sie z.B. bei oraler Verabreichung in Dosen von 20 bis 500 mg/kg an Ratten im Galactosamintest eine ausgeprägte leberschützende Wirkung und bei intravenöser Verabreichung in Dosen von 10 bis 300 mg/kg an Mäusen im α-Amanitintest eine ausgeprägte mortalitätssenkende Wirkung.

Diese Tests sind in der bereits angeführten Arbeit von G. Vogel et al., erschienen in Arzneimittelforschung, Band 25 (1975), auf den Seiten 82 bis 89 und 179 bis 188 beschrieben.

Im Galactosamintest erhielten Ratten in Gruppen zu je 15 Tieren 100 mg/kg der zu testenden Verbindung N-[5-(trans-2,3-Dihydro-7-methoxy-3-methyl-5-(E)-propenylbenzofuran-2-yl)-2-hydroxy-3-methoxyphenylmethyl]-N-(2-hydroxyäthyl)-N-methyl-ammoniumchlorid und zum Vergleich Silymarin in Traganthsuspension peroral verabreicht, eine Stunde später intraperitoneal 350 mg/kg Galactosamin. Hydrochlorid und 24 h später erfolgte Entnahme der Blutproben zur Bestimmung der Serumenzyme GOT und GPT.

| | GOT | GPT |
|---|---|---|
| Galactosamin-Hydrochlorid ohne Testverbindung | 572 | 578 |
| Kontrolle | 113 | 53 |
| N-[5-(trans-2,3-Dihydro-7-methoxy-3-methyl-5-(E)-propenylbenzofuran-2-yl)-2-hydroxy-3-methoxyphenylmethyl]-N-(2-hydroxyäthyl)-N-methylammoniumchlorid | 422 | 185 |
| Silymarin | 794 | 225 |

Besonders beim Enzym GPT war die Wirkung der getesteten neuen Verbindung hochsignifikant ($p < 0{,}01$) und deutlich besser als jene von Silymarin.

Im α-Amanitintest erhielten Mäuse in Gruppen zu je 20 Tieren 50 mg/kg N-[5-(trans-2,3-Dihydro-7-methoxy-3-methyl-5-(E)-propenylbenzofuran-2-yl)-2-hydroxy-3-methoxy-phenyl-methyl]-N-(2-hydroxyäthyl)-N-methylammoniumchlorid und zum Vergleich Silymarin intravenös verabreicht und eine Stunde später 0,7 mg/kg α-Amanitin intraperitoneal. Der Mortalitätsverlauf wurde 7 Tage beobachtet. Silymarin bewirkte keine Verringerung der Mortalitätsfälle gegenüber der Kontrollgruppe, bei N-[5-(trans-2,3-Dihydro-7-methoxy-3-methyl-5-(E)-propenylbenzofuran-2-yl)-2-hydroxy-3-methoxyphenylmethyl]-N-(2-hydroxyäthyl-N-methyl-ammoniumchlorid betrug die Mortalitätsrate hingegen nur 20 %.

Die toxikologischen Untersuchungen zeigen eine gute Verträglichkeit der nach dem erfindungsgemässen Verfahren hergestellten Verbindungen. So liegt z.B. die $LD_{50}$ bei Ratten nach peroraler Verabreichung für N-[5-(trans-2,3-Dihydro-7-methoxy-3-methyl-5-(E)-propenylbenzofuran-2-yl)-2-hydroxy-3-methoxyphenylmethyl]-N-(2-hydroxyäthyl)-N-methylammoniumchlorid zwischen 4 und 16 g/kg.

Die neuen Verbindungen enthaltende galenische Präparate sind daher wertvolle Heilmittel für die Behandlung von Leberschäden und zur Vorbeugung gegen ihre Entstehung.

Die pharmakologisch aktiven, nach dem erfindungsgemässen Verfahren hergestellten u.z. Teil neuen Verbindungen können in Form pharmazeutischer Präparate Verwendung finden, die sie in freier Form oder in Form ihrer Salze, besonders der therapeutisch verwendbaren Salze, in Mischung mit einem für die enterale oder parenterale Applikation geeigneten pharmazeutischen anorganischen oder organischen, festen oder flüssigen Trägermaterial enthalten. Für die Bildung derselben kommen solche Stoffe in Betracht, die mit dem Wirkstoff nicht reagieren, z.B. Wasser, Gelatine, Lactose, Stärke, Traganth, Stearylalkohol, Magnesiumstearat, Talk, pflanzliche Oele, Benzylalkohole, Propylenglykole, Vaseline und andere bekannte Arzneimittelträger. Die pharmazeutischen Präparate können z.B. als Tabletten, Dragées, Kapseln, Suppositorien, oder in flüssiger Form als Lösungen (z.B. Elixier oder Sirup), Suspension oder Emulsion vorliegen. Gegebenenfalls sind sie sterilisiert und/oder enthalten Hilfsstoffe, wie Konservierungsmittel, Stabilisatoren, Netz- oder Emulgiermittel, Lösungsvermittler oder Salze zur Veränderung des osmotischen Drucks oder Puffer. Sie können auch andere therapeutisch wertvolle Substanzen enthalten. Die pharmazeutischen Präparate werden nach üblichen Methoden gewonnen.

## Beispiel 1

Man hält 20,0 g trans-2,3-Dihydro-2-(4-hydroxy-3-methoxyphenyl)-7-methoxy-3-methyl-5-(E)-propenylbenzofuran und 16,0 g 3-Methyl-1,3-oxazolidin in 120 ml Aethanol 30 h bei 60 °C und entfernt anschliessend Lösungsmittel und überschüssiges 3-Methyl-1,3-oxazolidin, zuletzt im Vakuum. Der verbleibende Rückstand wird aus Methylenchlorid/Petroläther kristallisiert und gibt 19,8 g N-[5-(trans-2,3-Dihydro-7-methoxy-3-methyl-5-(E)-propenylbenzofuran-2-yl)-2-hydroxy-3-methoxyphenylmethyl]-N-(2-hydroxyäthyl)-N-methylamin, feine farblose Kristalle, Fp. 101-103 °C (Zers.).

$C_{24}H_{31}NO_5$ (413,31)
Ber. C 69,68 H 7,58 N 3,39
Gef. C 69,51 H 7,59 N 3,27

Hydrochlorid (mittels ätherischer Salzsäure) : feine schwach gelbliche hygroskopische Kristalle, F.p. 74 °C (Zers.).

$C_{24}H_{32}ClNO_5$ (449,77)
Ber. C 64,03 H 7,19 Cl 7,88 N 3,11
Gef. C 63,79 H 7,13 Cl 7,77 N 3,02

## Beispiel 2

Man hält 20,0 g (1α, 2β, 3α)-1-Aethyl-3-(4-hydroxy-3-methoxyphenyl)-6-methoxy-2-methyl-5-indanol und 21,6 g 3-Methyl-1,3-oxazolidin in 280 ml absolutem Alkohol 36 h bei 60 °C und entfernt anschliessend Lösungsmittel und überschüssiges 3-Methyl-1,3-oxazolidin, zuletzt im Vakuum. Nach Reinigung über eine chromatographische Säule erhält man 13,6 g N-[5-((1α, 2β, 3α)-1-Aethyl-5-hydroxy-6-methoxy-2-methylindan-3-yl)-2-hydroxy-3-methoxyphenylmethyl]-N-(2-hydroxyäthyl)-N(methylamin, farblose Kristalle, Fp. 141-143 °C.

$C_{24}H_{33}NO_5$ (415,33)
Ber. C 69,34 H 8,03 N 3,37
Gef. C 69,13 H 7,97 N 3,31

## Beispiel 3

Man hält 20,0 g trans-2,3-Dihydro-2-(4-hydroxy-3-methoxyphenyl)-7-methoxy-3-methyl-5-(E)-propenylbenzofuran und 14,4 g N-(2-Hydroxyäthyl)-1,3(oxazolidin in 240 ml absolutem Aethanol 48 h bei 70 °C und entfernt anschliessend Lösungsmittel und überschüssiges N-(2-Hydroxyäthyl)-1,3-oxazolidin, zuletzt im Vakuum. Digerieren des Rückstands mit Diäthyläther gibt 14,2 g N-[5-(trans-2,3-Dihydro-7-methoxy-3-methyl-5-(E)-propenyl-benzofuran-2-yl)-2-hydroxy-3-methoxy-phenylmethyl]-N,N-bis(2-hydroxyäthyl)amin, farblose Kristalle, Fp. 107 °C.

$C_{25}H_{33}NO_6$ (443,33)
Ber. C 67,67 H 7,52 N 3,16
Gef. C 67,35 H 7,61 N 3,22

## Beispiel 4

Man hält 20,0 g trans-2,3-Dihydro-2-(4-hydroxy-3-methoxyphenyl)-7-methoxy-3-methyl-5-(E)-propenylbenzofuran und 20,0 g 3-Benzyl-1,3-oxazolidin in 200 ml absolutem Aethanol 48 h bei 70 °C und entfernt anschliessend Lösungsmittel und überschüssiges 3-Benzyl-1,3-oxazolidin, zuletzt im Vakuum. Nach Reinigung über eine chromatographische Säule erhält man 17,2 g N-[5-(trans-2,3-Dihydro-7-methoxy-3-methyl-5-(E)-propenyl-benzofuran-2-yl)-2-hydroxy-3-methoxyphenylmethyl]-N-(2-hydroxyäthyl-N-benzylamin, ein farbloses Oel, das im Oelpumpenvakuum glasartig erstarrt.

$C_{30}H_{35}NO_5$ (489,37)
Ber. C 73,56 H 7,22 N 2,86
Gef. C 73,02 H 7,31 N 2,57

Hydrochlorid (mittels ätherischer Salzsäure) : feine hellgelbe hygroskopische Kristalle, Fp. 70 °C (Zers.).

$C_{30}H_{36}ClNO_5$ (525,82)
Ber. C 68,46 H 6,92 N 2,66 Cl 6,74
Gef. C 68,73 H 6,96 N 2,47 Cl 6,88

## Beispiel 5

Man hält 20,0 g (1α, 2β, 3α)-1-Aethyl-3-(4-hydroxy-3-methoxyphenyl)-6-methoxy-2-methyl-5-indanol

und 23,4 g 3-Butyl-1,3-oxazolidin in 250 ml absolutem Alkohol 48 h bei 65 °C und entfernt anschliessend das Lösungsmittel und überschüssiges 3-Butyl-1,3-oxazolidin, zuletzt im Vakuum. Nach Reinigung über eine chromatographische Säule und Kristallisation aus $CH_2Cl_2$/Benzin erhält man 14,7 g N-[5-((1α, 2β, 3α)-1-Aethyl-5-hydroxy-6-methoxy-2-methylindan-3-yl)-2-hydroxy-3-methoxyphenylmethyl]-N-(2-hydroxyäthyl)-N-butylamin, farblose Kristalle, Fp. 105-106 °C.

$C_{27}H_{39}NO_5$ (4,57,39)

| | | | |
|---|---|---|---|
| Ber. | C 70,84 | H 8,61 | N 3,06 |
| Gef. | C 70,88 | H 8,75 | N 3,01 |

Beispiel 6

Man hält 20,0 g trans-2,3-Dihydro-2-(4-hydroxy-3-methoxyphenyl)-7-methoxy-3-methyl-5-(E)-propenylbenzofuran und 32,8 g 2-(3-Chlorphenyl)-3-benzyl-1,3-oxazolidin in 260 ml absolutem Alkohol 60 h bei 70 °C und entfernt anschliessend das Lösungsmittel und überschüssiges 2-(3-Chlorphenyl)-3-benzyl-1,3-oxazolidin, zuletzt im Vakuum. Nach Reinigung über eine chromatographische Säule erhält man 9,8 g N-[5-(trans-2,3-Dihydro-7-methoxy-3-methyl-5-(E)-propenyl-benzofuran-2-yl)-2-hydroxy-3-methoxyphenylmethyl]-N-(1-hydroxy-1-phenyl-2-propyl)-N-methylamin, ein gelbes Oel.

$C_{36}H_{38}ClNO_5$

| | | | | |
|---|---|---|---|---|
| Ber. | C 72,02 | H 6,40 | N 2,33 | Cl 5,91 |
| Gef. | C 72,68 | H 65,7 | N 2,23 | Cl 5,98 |

Beispiel 7

Man hält 20,0 g trans-2,3-Dihydro-2-(4-hydroxy-3-methoxyphenyl)-7-methoxy-3-methyl-5-(E)-propenylbenzofuran und 21,3 g 3,4-Dimethyl-5-phenyl-1,3-oxazolidin in 200 ml absolutem Alkohol 60 h bei 70 °C und entfernt anschliessend das Lösungsmittel und überschüssiges 3,4-Dimethyl-5-phenyl-1,3-oxazolidin, zuletzt im Vakuum. Nach Reinigung über eine chromatographische Säule erhält man 9,1 g N-[5-(trans-2,3-Dihydro-7-methoxy-3-methyl-5-(E)-propenyl-benzofuran-2-yl)-2-hydroxy-3-methoxyphenylmethyl]-N-(1-hydroxy-1-phenyl-2-propyl)-N-methylamin, ein gelbes Oel.

$C_{31}H_{37}NO_5$ (503,37)

| | | | |
|---|---|---|---|
| Ber. | C 73,90 | H 7,42 | N 2,78 |
| Gef. | C 74,12 | H 7,49 | N 2,67 |

Beispiel 8

Man hält 20,0 g trans-2,3-Dihydro-2-(4-hydroxy-3-methoxyphenyl)-7-methoxy-3-methyl-5-(E)-propenylbenzofuran und 17,2 g 2-Isopropyl-3-äthyl-1,3-oxazolidin in 180 ml absolutem Aethanol 36 h bei 60 °C und entfernt dann das Lösungsmittel und überschüssiges 2-Isopropyl-1,3-oxazolidin im Vakuum. Nach Reinigung über eine chromatographische Säule erhält man 10,6 g N-[5-(trans-2,3-Dihydro-7-methoxy-3-methyl-5-(E)-propenyl-benzofuran-2-yl)-2-hydroxy-3-methoxyphenyl-(isopropyl)-methyl]-N-(2-hydroxyäthyl)-N-äthylamin, ein hellbraunes Oel.

$C_{28}H_{39}NO_5$ (469,39)

| | | | |
|---|---|---|---|
| Ber. | C 71,58 | H 8,39 | N 2,98 |
| Gef. | C 71,82 | H 8,51 | N 2,90 |

Beispiel 9

Man Hält 20,0 g trans-2,3-Dihydro-2-(4-hydroxy-3-methoxyphenyl)-7-methoxy-3-methyl-5-(E)-propenylbenzofuran und 13,6 g 3-Allyl-1,3-oxazolidin in 240 ml absolutem Alkohol 48 h bei 70 °C und entfernt dann das Lösungsmittel und überschüssiges 3-Allyl-1,3-oxazolidin im Vakuum. Nach Reinigung über eine chromatographische Säule erhält man 14,4 g N-[5-(trans-2,3-Dihydro-7-methoxy-3-methyl-5-(E)-propenylbenzofuran-2-yl)-2-hydroxy-3-methoxyphenylmethyl]-N-(2-hydroxyäthyl)-N-allylamin, ein farbloses Oel.

$C_{26}H_{33}NO_5$ (439,33)

| | | | |
|---|---|---|---|
| Ber. | C 71,02 | H 7,59 | N 3,19 |
| Gef. | C 70,92 | H 7,61 | N 3,16 |

Beispiel 10

Man hält 20,0 g (1α, 2β, 3α)-1-Aethyl-3-(4-hydroxy-3-methoxyphenyl)-6-methoxy-2-methyl-5-indanol und 31,9 g 3,4-Dimethyl-5-phenyl-1,3-oxazolidin in 250 ml absolutem Alkohol 60 h bei 70 °C und entfernt dann das Lösungsmittel und überschüssiges 3,4-Dimethyl-5-phenyl-1,3-oxazolidin im Vakuum. Nach Reinigung über eine chromatographische Säule erhält man 8,9 g N-[5-((1α, 2β, 3α)-1-Aethyl-5-hydroxy-6-methoxy-2-methylindan-3-yl)-2-hydroxy-3-methoxyphenylmethyl]-N-(1-hydroxy-1-phenyl-2-propyl)-N-methylamin, ein hellbraunes Oel.

$C_{31}H_{39}NO_5$ (505,39)
Ber. C 73,61 H 7,79 N 2,77
Gef. C 73,95 H 7,87 N 2,64

Beispiel 11

Man hält 20,0 g trans-2,3-Dihydro-2-(4-hydroxy-3-methoxyphenyl)-7-methoxy-3-methyl-5-(E)-propenylbenzofuran und 15,5 g 3-Butyl-1,3-oxazolidin in 180 ml absolutem Alkohol 36 h bei 70 °C und entfernt dann das Lösungsmittel und überschüssiges 3-Butyl-1,3-oxazolidin im Vakuum. Nach Reinigung über eine chromatographische Säule erhält man 15,8 g N-[5-(trans-2,3-Dihydro-7-methoxy-3-methyl-5-(E)-propenyl-benzofuran-2-yl)-2-hydroxy-3-methoxyphenylmethyl]-N-(2-hydroxyäthyl)-N-butylamin, ein farbloses Oel.

$C_{27}H_{37}NO_5$ (455,37)
Ber. C 71,15 H 8,21 N 3,07
Gef. C 71,25 H 8,15 N 3,00

Beispiel 12

Herstellung von 10 000 Kapseln mit einem Gehalt von je 100 mg der aktiven Substanz :

Bestandteile :

| | |
|---|---|
| N-[5-(trans-2,3-Dihydro-7-methoxy-3-methyl-5-(E)-propenylbenzofuran-2-yl)-2-hydroxy-3-methoxy phenylmethyl]-N-(2-hydroxyäthyl)-N-methylamin | 1 000 g |
| Milchzucker | 2 800 g |
| Talkpulver | 200 g |

Verfahren :

Die pulvrigen Bestandteile werden mit einem Sieb von 0,6 mm Maschenweite gesiebt. Dann wird der Wirkstoff in einem Mischer zuerst mit dem Talkpulver und dann mit dem Milchzucker homogenisiert. Unter Verwendung einer Füllmaschine werden Gelatinekapseln entsprechender Grösse mit je 400 mg Gemisch gefüllt.

Beispiel 13

Herstellung von 10 000 ml Injektionslösung zur Abfüllung in Ampullen :

Bestandteile :

| | |
|---|---|
| N-[5-(trans-2,3-Dihydro-7-methoxy-3-methyl-5-(e)-propenylbenzofuran-2-yl)-2-hydroxy-3-methoxyphenylmethyl]-N-(2-hydroxyäthyl)-N-methylammoniumchlorid | 600 g |
| Natriumchlorid | 10 g |
| Aqua pro injectione | ad 10 000 ml |

Verfahren :

Nach dem Auflösen der Bestandteile filtriert man durch eine Glasfilternutsche der Durchlässigkeit G 3 und füllt hierauf unter Stickstoff in Ampullen ab, zur Verwendung für i.v.-Injektionen in solche von 2 ml Inhalt, bei Verwendung als Zusatz von Infusionen in solche zu 5 ml Inhalt.

Beispiel 14

Herstellung von 10 000 Tablennen mit einem Gehalt von je 50 mg der aktiven Substanz :

Bestandteile :

| | |
|---|---|
| N-[5-((1α, 2β, 3α)-1-Äethyl-5-hydroxy-6-methoxy-2-methylindan-3-yl)-2-hydroxy-3-methoxyphenylmethyl]-N-(2-hydroxyäthyl)-N-methylamin | 500 g |
| Milchzucker | 1 700 g |
| Maisstärke | 90 g |
| Polyäthylenglykol 6 000 | 90 g |
| Talkpulver | 90 g |
| Magnesiumstearat | 30 g |

Verfahren :

Die pulvrigen Bestandteile werden mit einem Sieb von 0,6 mm Maschenweite gesiebt. Dann wird der Wirkstoff mit Milchzucker, Talk, Magnesiumstearat und der Hälfte der Stärke in einem geeigneten Mischer vermischt. Die andere Hälfte der Stärke wird in 50 ml Wasser suspendiert und die Suspension zu einer 80° heissen Lösung des Polyäthylenglykols in 190 ml Wasser gegeben. Die auf diese Weise erhaltene Paste wird zu der Mischung der pulvrigen Bestandteile gegeben und man granuliert, gegebenenfalls unter Zugabe einer weiteren Wassermenge. Das Granulat wird 12 h bei 30° getrocknet, durch ein Sieb 1,2 mm Maschenweite getrieben und zu Tabletten von 7 mm Durchmesser verpresst.

Beispiel 15

Herstellung von 10 000 Kapseln mit einem Gehalt von je 50 mg der aktiven Substanz :

Bestandteile :

| | |
|---|---|
| N-[5-(trans-2,3-Dihydro-7-methoxy-3-methyl-5-(E)-propenylbenzofuran-2-yl)-2-hydroxy-3-methoxyphenyl-methyl]-N-(2-hydroxyäthyl)-benzylammoniumchlorid | 500 g |
| Milchzucker | 2 800 g |
| Talkpulver | 200 g |

Verfahren :

Nach im wesentlichen der gleichen Vorgangsweise wie bei Beispiel 12 füllt man unter Verwendung einer Füllmaschine Gelatinekapseln entsprechender Grösse mit je 350 mg Gemisch.

Beispiel 16

Herstellung von 10 000 Tabletten mit einem Gehalt von je 70 mg der aktiven Substanz :

Bestandteile :

| | |
|---|---|
| N-[5-((1α, 2β, 3α)-1-Aethyl-5-hydroxy-6-methoxy-2-methylindan-3-yl)-2-hydroxy-3-methoxyphenylmethyl]-N-(2-hydroxyäthyl)-N-butylamin | 700 g |
| Maisstärke | 1 000 g |
| Magnesiumstearat | 90 g |
| Mannit | 100 g |
| Mikrokristalline Kieselsäure | 100 g |
| Lactose | 350 g |

Verfahren :

In analoger Vorgangsweise wie bei Beispiel 14 werden die Bestandteile gesiebt und mit Wasser zu einer festen Paste verarbeitet, die man granuliert und nach Trocknung und Sieben zu Tabletten verpresst.

**Ansprüche**

1. Verfahren zum Herstellen von Derivaten von Dimeren des Isoeugenols der Formeln

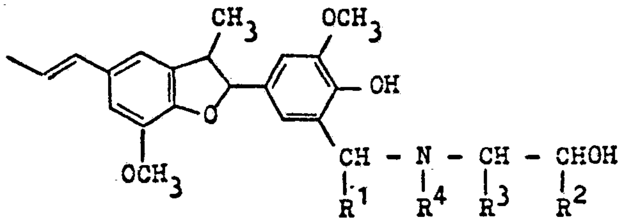

(IV)

und

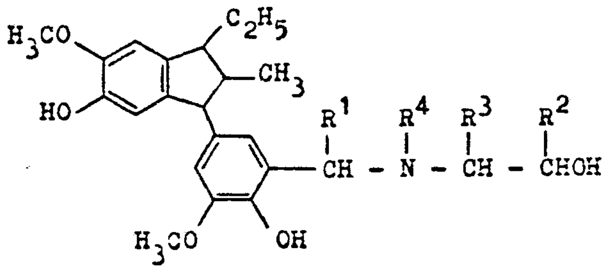

(V)

worin

$R^1$ Wasserstoff, einen verzweigten oder unverzweigten Alkalrest mit ein bis vier Kohlenstoffatomen, ausgenommen die tert.-Butylgruppe, oder einen gegebenenfalls durch Halogen substituierten Phenylrest,

$R^2$ Wasserstoff oder einen Phenylrest,

$R^3$ Wasserstoff oder die Methylgruppe, und

$R^4$ eine verzweigte oder unverzweigte Alkalgruppe mit ein bis vier Kohlenstoffatomen, die eine Hydroxygruppe tragen kann, oder die Allyl- oder Benzylgruppe bedeuten,

und ihren Salzen mit Säuren, dadurch gekennzeichnet, dass man ein Dimeres von Isoeugenol der Formel

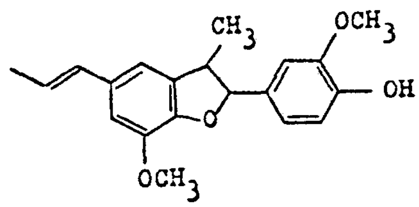

(I, Dehydrodiisoeugenol)

bzw.

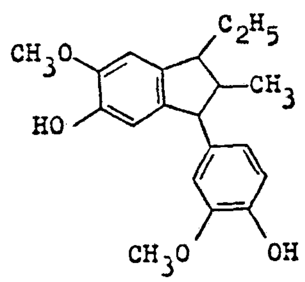

(II, Diisoeugenol)

mit einem Oxazolidin der Formel

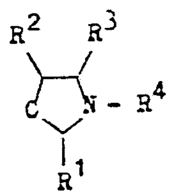

(III)

umsetzt, worin die Symbole die obige Bedeutung haben, und gegebenenfalls die erhaltenen Verbindungen in ihre Salze mit Säuren Überführt.

2. Verfahren nach Anspruch 1, dadurch gekennzeichnet, dass man von Dehydrodiisoeugenol und 3-Methyl-1,3-oxazolidin ausgeht und N-[5-(trans-2,3-Dihydro-7-methoxy-3-methyl-5-(E)-propenylbenzofuran-2-yl)-2-hydroxy-3-methoxyphenylmethyl]-N-(2-hydroxyäthyl)-N-methylamin herstellt.

3. Verfahren nach Anspruch 1, dadurch gekennzeichnet, dass man von Diisoeugenol und 3-Methyl-1,3-oxazolidin ausgeht und N-[5-(1α, 2β, 3α)-1-Aethyl-5-hydroxy-6-methoxy-2-methylindan-3-yl)-2-hydroxy-3-methoxyphenylmethyl]-N-(2-hydroxyäthyl)-N-methylamin herstellt.

4. Verfahren nach Anspruch 1, dadurch gekennzeichnet, dass man von Dehydrodiisoeugenol und N-(2-Hydroxyäthyl)-1,3-oxazolidin ausgeht und N-[5-(trans-2,3-Dihydro-7-methoxy-3-methyl-5-(E)-propenylbenzofuran-2-yl)-2-hydroxy-3-methoxyphenylmethyl]-N,N-bis(2-hydroxyäthyl)amin herstellt.

5. Verfahren nach Anspruch 1, dadurch gekennzeichnet, dass man von Dehydrodiisoeugenol und 3-Benzyl-1,3-oxazolidin ausgeht und N-[5-(trans-2,3-Dihydro-7-methoxy-3-methyl-5-(E)-propenylbenzofuran-2-yl)-2-hydroxy-3-methoxyphenylmethyl]-N-(2-hydroxyäthyl)-N-benzylamin herstellt.

6. Verfahren nach Anspruch 1, dadurch gekennzeichnet, dass man von Diisoeugenol und 3-Butyl-1,3-oxazolidin ausgeht und N-[5-((1α, 2β, 3α)-1-Aethyl-5-hydroxy-6-methoxy-2-methylindan-3-yl)-2-hydroxy-3-methoxyphenylmethyl]-N-(2-hydroxyäthyl)-N-butylamin herstellt.

7. Verfahren nach Anspruch 1, dadurch gekennzeichnet, dass man von Dehydrodiisoeugenol und 2-(3-Chlorphenyl)-3-benzyl-1,3-oxazolidin ausgeht und N-[5-(trans-2,3-Dihydro-7-methoxy-3-methyl-5-(E)-propenylbenzofuran-2-yl)-2-hydroxy-3-methoxyphenyl-(3-chlorphenyl)-methyl]-N-(2-hydroxyäthyl)-N-benzylamin herstellt.

8. Verfahren nach Anspruch 1, dadurch gekennzeichnet, dass man von Dehydrodiisoeugenol und 3,4-Dimethyl-5-phenyl-1,3-oxazolidin ausgeht und N-[5-(trans-2,3-Dihydro-7-methoxy-3-methyl-5-(E)-propenyl-benzofuran-2-yl)-2-hydroxy-3-methoxyphenylmethyl]-N-(1-hydroxy-1-phenyl-2-propyl)-N-methylamin herstellt.

9. Verfahren nach Anspruch 1, dadurch gekennzeichnet, dass man von Dehydrodiisoeugenol und 2-Isopropyl-3-äthyl-1,3-oxazolidin ausgeht und N-[5-(trans-2,3-Dihydro-7-methoxy-3-methyl-5-(E)-propenylbenzofuran-2-yl)-2-hydroxy-3-methoxyphenyl-(isopropyl)-methyl]-N-(2-hydroxyäthyl)-N-äthylamin herstellt.

10. Verfahren nach Anspruch 1, dadurch gekennzeichnet, dass man von Dehydrodiisoeugenol und 3-Allyl-1,3-oxazolidin ausgeht und n-[5-(trans-2,3-Dihydro-7-methoxy-3-methyl-5-(E)-propenylbenzofuran-2-yl)-2-hydroxy-3-methoxyphenylmethyl]-N-(2-hydroxyäthyl)-N-allylamin herstellt.

11. Verfahren nach Anspruch 1, dadurch gekennzeichnet, dass man von Diisoeugenol und 3,4-Dimethyl-5-phenyl-1,3-oxazolidin ausgeht und N-[5-((1α, 2β, 3α)-1-Aethyl-5-hydroxy-6-methoxy-2-methylindan-3-yl)-2-hydroxy-3-methoxyphenylmethyl]-N-(1-hydroxy-1-phenyl-2-propyl)-N-methylamin herstellt.

12. Verfahren nach Anspruch 1, dadurch gekennzeichnet, dass man von Dehydrodiisoeugenol und 3-Butyl-1,3-oxazolidin ausgeht und N-[5-(trans-2,3-Dihydro-7-methoxy-3-methyl-5-(E)-propenylbenzofuran-2-yl)-2-hydroxy-3-methoxyphenylmethyl]-N-(2-hydroxyäthyl)-N-butylamin herstellt.

13. Verfahren nach einem der Ansprüche 1 bis 12, dadurch gekennzeichnet, dass man als Lösungsmittel Methanol, Aethanol, 2-Propanol, Diäthyläther, Diisopropyläther, Dioxan, Tetrahydrofuran, Acetonitril oder Propionitril einsetzt.

**Claims**

1. Process for the preparation of derivatives of isoeigenol dimers of the formulas

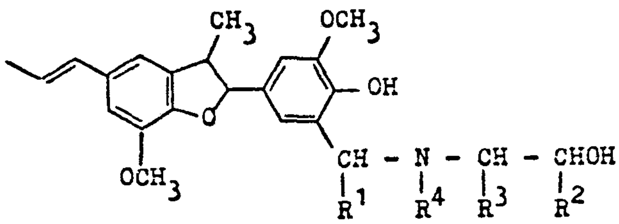

(IV)

and

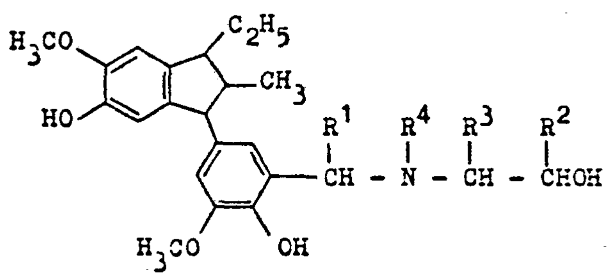

(V)

wherein

$R^1$ is hydrogen, a branched or unbranched alkyl group with one to four carbon atoms, except the tert. butyl group, or a phenyl group possibly substituted by halogen,

$R^2$ is hydrogen or a phenyl group

$R^3$ is hydrogen or the methyl group, and

$R^4$ is a branched or unbranched alkyl group with one to four carbon atoms, which may carry a hydroxy group, or the allyl or benzyl group,
as well as their salts with acids, characterized in that a dimer of isoeugenol of the formula

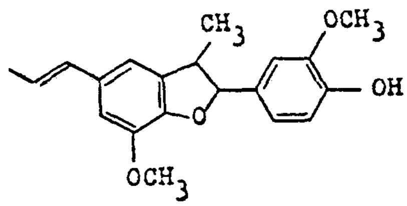

(I, Dehydrodiisoeugenol)

or

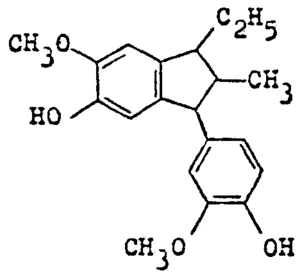

(II, Diisoeugenol)

respectively is reacted with an oxazolidine of formula

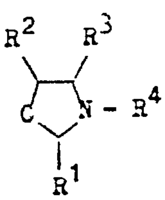

(III)

wherein the symbols have the above meaning, and the obtained compounds are possibly transformed into their salts with acids.

2. Process according to claim 1, characterized in that one starts from dehydrodiisoeugenol and 3-methyl-1,3-oxazolidine for preparing N-[5-(trans-2,3-dihydro-7-methoxy-3-methyl-5-(E)-propenylbenzofuran-2-yl-2-hydroxy-3-methoxyphenylmethyl]-N-(2-hydroxyethyl)-N-methylamine.

3. Process according to claim 1, characterized in that one starts from diisoeugenol and 3-methyl-1,3-oxazolidine for preparing N-[5-(1 alpha, 2 beta, 3 alpha)-1-ethyl-5-hydroxy-6-methoxy-2-methylindan-3-yl)-2-hydroxy-3-methoxyphenylmethyl]-N-(2-hydroxyethyl)-N-methylamine.

4. Process according to claim 1, characterized in that one starts from dehydrodiisoeugenol and N-(2-hydroxyethyl)-1,3-oxazolidine for preparing N-[5-(trans-2,3-dihydro-7-methoxy-3-methyl-5-(E)-propenylbenzofuran-2-yl)-2-hydroxy-3-methoxyphenylmethyl]-N,N-bis(2-hydroxyethyl)amine.

5. Process according to claim 1, characterized in that one starts from dehydrodiisoeugenol and 3-benzyl-1,3-oxazolidine for preparing N-[5-(trans-2,3-dihydro-7-methoxy-3-methyl-5-(E)-propenylbenzofuran-2-yl)-2-hydroxy-3-methoxyphenylmethyl]-N-(2-hydroxyethyl)-N-benzylamine.

6. Process according to claim 1, characterized in that one starts from diisoeugenol and 3-butyl-1,3-oxazolidine for preparing N-[5-((1 alpha, 2 beta, 3 alpha)-1-ethyl-5-hydroxy-6-methoxy-2-methylindan-3-yl)-2-hydroxy-3-methoxyphenylmethyl]-N-(2-hydroxyethyl)-N-butylamine.

7. Process açcording to claim.1, characterized in that one starts from dehydrodiisoeugenol and 2-(3-chlorophenyl)-3-benzyl-1,3-oxazolidine for preparing N-[5-(trans-2,3-dihydro-7-methoxy-3-methyl-5-(E)-propenylbenzofuran-2-yl)-2-hydroxy-3-methoxyphenyl-(3-chlorophenyl)-methyl]-N-(2-hydroxyethyl)-N-benzylamine.

8. Process according to claim 1, characterized in that one starts from dehydrodiisoeugenol and 3,4-dimethyl-5-phenyl-1,3-oxazolidine for preparing N-[5-(trans-2,3-dihydro-7-methoxy-3-methyl-5-(E)-propenylbenzofuran-2-yl)-2-hydroxy-3-methoxyphenylmethyl]-N-(1-hydroxy-1-phenyl-2-propyl)-N-methylamine.

9. Process according to claim 1, characterized in that one starts from dehydrodiisoeugenol and 2-isopropyl-3-ethyl-1,3-oxazolidine for preparing N-[5-(trans-2,3-dihydro-7-methoxy-3-methyl-5-(E)-prope-

nylbenzofuran-2-yl)-2-hydroxy-3-methoxyphenyl-(isopropyl)-methyl]-N-(2-hydroxyethyl)-N-ethylamine.

10. Process according to claim 1, characterized in that one starts from dehydrodiisoeugenol and 3-allyl-1,3-oxazolidine for preparing N-[5-(trans-2,3-dihydro-7-methoxy-3-methyl-5-(E)-propenylbenzofuran-2-yl)-2-hydroxy-3-methoxyphenylmethyl]-N-(2-hydroxyethyl)-N-allylamine.

11. Process according to claim 1, characterized in that one starts from diisoeugenol and 3,4-dimethyl-5-phenyl-1,3-oxazolidine for preparing N-[5-((1alpha, 2beta, 3alpha)-1-ethyl-5-hydroxy-6-methoxy-2-methylindan-3-yl)-2-hydroxy-3-methoxyphenylmethyl]-N-(1-hydroxy-1-phenyl-2-propyl)-N-methylamine.

12. Process according to claim 1, characterized in that one starts from dehydrodiisoeugenol and 3-butyl-1,3-oxazolidine for preparing N-[5-(trans-2,3-dihydro-7-methoxy-3-methyl-5-(E)-propenylbenzofuran-2-yl)-2-hydroxy-3-methoxyphenylmethyl]-N-(2-hydroxyethyl)-N-butylamine.

13. Process according to one of claims 1 to 12, characterized in that methanol, ethanol, 2-propanol, diethyl ether, diisopropyl ether, dioxan, tetrahydrofuran, acetonitrile or propionitrile is used as a solvant.

**Revendications**

1. Procédé de préparation de dérivés de dimères de l'isoeugénol de formules :

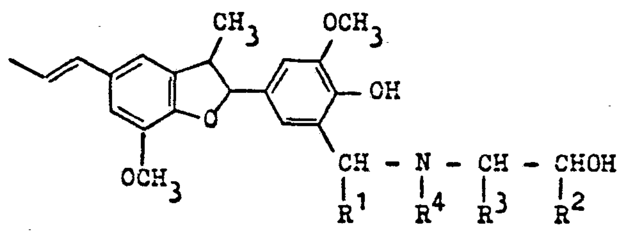

(IV)

et

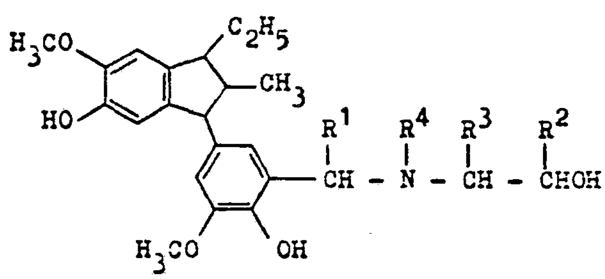

(V)

dans lesquelles :

$R^1$ représente l'hydrogène, un groupe alcoyle ramifié ou non ramifié avec 1 à 4 atomes de carbone, à l'exception du groupe tert.-butyle, ou un groupe phényle éventuellement substitué par de l'halogène,

$R^2$ représente l'hydrogène ou un groupe phényle,

$R^3$ représente l'hydrogène ou le groupe méthyle, et

$R^4$ est un groupe alcoyle ramifié ou non ramifié avec 1 à 4 atomes de carbone, pouvant porter un groupe hydroxy, ou le groupe allyle ou benzyle.

et de leurs sels avec des acides, caractérisé en ce qu'on fait réagir un dimère d'isoeugénol de formule :

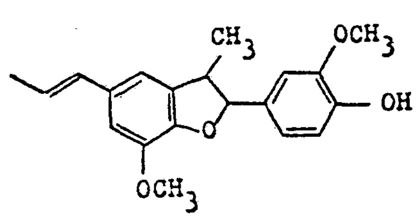

(I, déshydrodiisoeugénol)

ou

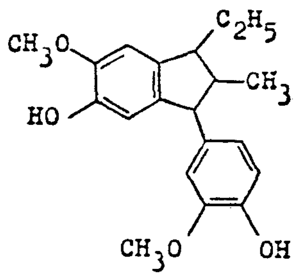

(II, diisoeugénol)

respectivement, avec une oxazolidine de formule :

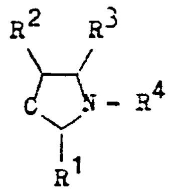

(III)

dans laquelle les symboles ont la signification mentionnée ci-dessus, et en ce qu'on transforme, le cas échéant, les composés obtenus en leurs sels avec des acides.

2. Procédé selon la revendication 1, caractérisé en ce qu'on part du déshydrodiisoeugénol et de la 3-méthyl-1,3-oxazolidine et prépare la N-[5-(trans-2,3-dihydro-7-méthoxy-3-méthyl-5-(E)-propénylbenzofuranne-2-yl)-2-hydroxy-3-méthoxyphénylméthyl]-N-(2-hydroxyéthyl)-N-méthylamine.

3. Procédé selon la revendication 1, caractérisé en ce qu'on part du diisoeugénol et de la 3-méthyl-1,3-oxazolidine et prépare la N-[5-(1α, 2β, 3α)-1-éthyl-5-hydroxy-6-méthoxy-2-méthylindane-3-yl)-2-hydroxy-3-méthoxyphénylméthyl]-N-(2-hydroxyéthyl)-N-méthylamine.

4. Procédé selon la revendication 1, caractérisé en ce qu'on part du déshydrodiisoeugénol et de la N-(2-hydroxyéthyl)-1,3-oxazolidine et prépare la N-[5-(trans-2,3-dihydro-7-méthoxy-3-méthyl-5-(E)-propénylbenzofuranne-2-yl)-2-hydroxy-3-méthoxyphénylméthyl]-N,N-bis(2-hydroxyéthyl)amine.

5. Procédé selon la revendication 1, caractérisé en ce qu'on part du déshydrodiisoeugénol et de la 3-benzyl-1,3-oxazolidine et prépare la N-[5-(trans-2,3-dihydro-7-méthoxy-3-méthyl-5-(E)-propénylbenzofuranne-2-yl)-2-hydroxy-3-méthoxyphénylméthyl]-N-(2-hydroxyéthyl)-N-benzylamine.

6. Procédé selon la revendication 1, caractérisé en ce qu'on part du diisoeugénol et de la 3-butyl-1,3-oxazolidine et prépare la N-[5-((1α, 2β, 3α)-1-éthyl-5-hydroxy-6-méthoxy-2-méthylindane-3-yl)-2-hydroxy-3-méthoxyphénylméthyl]-N-(2-hydroxyéthyl)-N-butylamine.

7. Procédé selon la revendication 1, caractérisé en ce qu'on part du déshydrodiisoeugénol et de la 2-(3-chlorophényl)-3-benzyl-1,3-oxazolidine et prépare la N-[5-(trans-2,3-dihydro-7-méthoxy-3-méthyl-5-(E)-propénylbenzofuranne-2-yl)-2-hydroxy-3-méthoxyphényl-(3)-chlorophényl)-méthyl]-N-(2-hydroxyéthyl)-N-benzylamine.

8. Procédé selon la revendication 1, caractérisé en ce qu'on part du déshydrodiisoeugénol et de la 3,4-diméthyl-5-phényl-1,3-oxazolidine et prépare la N-[5-(trans-2,3-dihydro-7-méthoxy-3-méthyl-5-(E)-propénylbenzofuranne-2-yl)-2-hydroxy-3-méthoxyphénylméthyl]-N-(1-hydroxy-1-phényl-2-propyl)-N-méthylamine.

9. Procédé selon la revendication 1, caractérisé en ce qu'on part du déshydrodiisoeugénol et de la 2-isopropyl-3-éthyl-1,3-oxazolidine et prépare la N-[5-(trans-2,3-dihydro-7-méthoxy-3-méthyl-5-(E)-propénylbenzofuranne-2-yl)-2-hydroxy-3-méthoxyphényl-(isopropyl)-méthyl]-N-(2-hydroxyéthyl)-N-éthylamine.

10. Procédé selon la revendication 1, caractérisé en ce qu'on part du déshydrodiisoeugénol et de la 3-allyl-1,3-oxazolidine et prépare la N-[5-(trans-2,3-dihydro-7-méthoxy-3-méthyl-5-(E)-propénylbenzofuranne-2-yl)-2-hydroxy-3-méthoxyphénylméthyl]-N-(2-hydroxyéthyl)-N-allylamine.

11. Procédé selon la revendication 1, caractérisé en ce qu'on part du diisoeugénol et de la 3,4-diméthyl-5-phényl-1,3-oxazolidine et prépare la N-[5-((1α, 2β, 3α)-1-éthyl-5-hydroxy-6-méthoxy-2-méthylindane-3-yl)-2-hydroxy-3-méthoxyphénylméthyl]-N-(1-hydroxy-1-phényl-2-propyl)-N-méthylamine.

12. Procédé selon la revendication 1, caractérisé en ce qu'on part du déshydrodiisoeugénol et de la 3-butyl-1,3-oxazolidine et prépare la N-[5-(trans-2,3-dihydro-7-méthoxy-3-méthyl-5-(E)-propénylbenzofuranne-2-yl)-2-hydroxy-3-méthoxyphénylméthyl]-N-(2-hydroxyéthyl)-N-butylamine.

13. Procédé selon l'une des revendications 1 à 12, caractérisé en ce qu'on utilise comme solvant le méthanol, l'éthanol, le 2-propanol, l'éther diéthylique, l'éther diisopropylique, le dioxanne, le tétrahydrofuranne, l'acétonitrile ou le propionitrile.